# EUROPÄISCHE PATENTSCHRIFT

(11) **EP 2 338 443 B1**
(45) Veröffentlichungstag und Bekanntmachung des Hinweises auf die Patenterteilung: **14.12.2016**
(21) Anmeldenummer: 10405242.8
(22) Anmeldetag: 20.12.2010
(51) Int. Cl.: A61F 2/34, A61F 2/30, A61F 2/00

(54) **Künstliche Hüftgelenkpfanne zur unzementierten Verankerung**
Artificial hip joint socket for uncemented anchoring
Cotyle d'articulation de la hanche artificielle destinée à l'ancrage non cimenté

(30) Priorität: 23.12.2009 CH 19842009
(43) Veröffentlichungstag der Anmeldung: 29.06.2011
(73) Patentinhaber: Atesos medical AG, 5000 Aarau (CH)
(72) Erfinder: Moser, Walter, 5018 Erlinsbach (CH)
(74) Vertreter: Schollweck, Susanne

(56) Entgegenhaltungen:
- EP-A1- 0 358 345
- EP-A1- 0 639 356
- EP-A2- 1 179 324
- WO-A1-97/39702
- WO-A1-97/42913
- WO-A2-02/064066
- DE-A1-102006 017 473
- FR-A1- 2 897 528
- US-A- 5 755 799

## Beschreibung

Die Erfindung betrifft eine metallische Schale gemäss dem Oberbegriff des Anspruches 1, zur zementfreien Verankerung im menschlichen Becken, in welche ein kalottenförmiger Lagerkörper eingesetzt wird, der als Artikulationspartner für einen kugelförmigen Gelenkkopf dient, welcher am oberen Ende des Oberschenkels angebracht ist.

Künstliche Hüftpfannen sind in verschiedenen Ausführungsvarianten bekannt. Neben einem kleineren Anteil Hüftpfannen, welche mittels Knochenzement verankert werden, wird der weitaus grössere Anteil so hergestellt und implantiert, dass eine biologische Fixation erreicht wird. Dabei wird durch eine geeignete Formgebung, geeignete Werkstoffe und Oberflächengestaltung und eine geeignete Operationstechnik die dauerhaft feste Verbindung mit dem Knochen angestrebt. Bezüglich der äusseren Form sind in der Praxis konisch geformte Pfannen und sphärische Pfannen gebräuchlich. Die Auswahl des einen oder des anderen Pfannentyp erfolgt je nach Indikationsstellung und Erfahrungen des behandelnden Arztes. Beide Pfannentypen umfassen jeweils eine metallische Aussenschale, hergestellt aus einem biologisch und mechanisch geeigneten Metallwerkstoff, und einem in der Aussenschale meist während der Operation zu montierenden Einsatz (Inlay) als Lagerkörper. Der Lagerkörper wird üblicherweise aus einem tribologisch geeigneten Werkstoff, z.B. aus Keramik, Kunststoff wie zum Beispiel Polyethylen (ultrahochmolekulares Polyethylen) oder zur Artikulation mit der Gelenkkugel geeigneten Metall hergestellt. Für beide Bauformen der Verankerungsschale, konisch und sphärisch, werden meist Titanwerkstoffe, welcher als Rein- Titan mit unterschiedlicher Härte und Festigkeit oder als Titanlegierung verfügbar sind, verwendet. Diese Werkstoffe sind bezüglich ihrer guten Verträglichkeit und der direkten Besiedelung mit Knochengewebe geeignet um mechanisch belastbare Verbindungen zwischen Implantat und Skelett zu erzeugen.

Derartige zweiteilige Konstruktionen von künstlichen Gelenkpfannen haben sich aufgrund ihrer gezielt auf die Anforderungen abstimmbaren Eigenschaften der Aussenschale und des Inlays als sehr geeignet und verlässlich erwiesen. So kann in der Planungsphase oder während der Operation aus einer Reihe von Inlays das für die gegebenen Patientenbedingungen am besten geeignete ausgewählt werden.

Im Folgenden soll vor allem auf sphärische Pfannen eingegangen werden. Ein Vorteil bei der Verwendung der sphärischen Pfannen liegt darin, dass für die Implantation beim Fräsvorgang etwas weniger Knochenvolumen zu entfernen ist als bei anders geformten, z.B. bei konischen Pfannen vergleichbarer Grösse. Eine Forderung, welche auch in einschlägigen Industrienormen festgeschrieben ist, ist eine minimale Wandstärke des Inlays" welches gegen die Hüftkugel artikuliert. Unter Berücksichtigung dieser Anforderung ergibt sich automatisch eine sphärische Pfannenaussenform. Mit sphärischen Pfannen kann also unter minimaler Knochenresektion ein bezüglich Sicherheit und Verschleiss optimiertes Implantat am besten verwirklicht werden. Zusätzlich ist bei sphärischen Pfannen die Implantationsorientierung der halbkugelförmigen Verankerungsschale innerhalb des kugelkalottenförmig vorgefrästen knöchernen Lagers frei wählbar. Der Operateur kann die Implantationsorientierung ggf. auch nach Fertigstellung aller Knochenbearbeitungsschritte den Gegebenheiten z.B. der Weichteilsituation anpassen. Dies wird insbesondere dann notwendig, wenn beim Test des Kunstgelenkes mit Probekomponenten Tendenzen zur Exartikulation von Pfanne und Gelenkkugel erkennbar werden, oder wenn Tendenzen zum Kontakt zwischen dem Rand des Inlays mit dem Zapfen, welcher die Prothesenkugel trägt erkennbar werden.

Bei der Präparation des knöchernen Lagers für sphärische Pfannen ist die Präzision des Knochenlagers deutlich weniger vom manuellen Geschick des Operateurs abhängig, als dies bei konischen Pfannen der Fall ist. Insbesondere muss während des Fräsens die Richtung nicht so genau eingehalten werden.

Voraussetzung für eine biologische Verbindung zwischen Implantat und Knochen ist eine geeignete Oberfläche und die sichere primär stabile Fixierung der Aussenschale im gefrästen knöchernen Lager. Diese Fixierung muss so stabil sein, dass keine Relativbewegungen zwischen Implantatoberfläche und Knochen auftreten. Zur Erreichung der Primärstabilität wird das halbschalenförmige Knochenlager mit einem gezielten Untermass gegenüber der Aussenschale hergestellt und diese dann in dieses untermassige Lager impaktiert. dabei verklemmt sich die Aussenschale im knöchernen Lager. Unterstütz wird diese Primärstabilität durch Verklemmung durch eine rau gestaltete Oberfläche und in einigen Ausführungen durch klein dimensionierte Geometrieelemente, welche in die Knochenstruktur eindringen.

Beckenimplantate, welche mittels Verklemmung einer sphärischen Halbschale im untermassig gefrästen Knochen verankert werden sind z.B. in WO 2007/118553 und in EP 1 179 324 A1 beschrieben. Die Verankerungsschale wird bei der Implantation mittels Hammerschlägen in das sich elastisch deformierende knöcherne Lager getrieben. Die Verklemmwirkung wird dabei durch eine deutlich aufgeraute Oberfläche unterstützt, erzeugt durch mechanisches Aufrauen der Oberfläche oder durch den Auftrag einer rauen Struktur, z.B. durch ein thermisches Spritzverfahren. In der sekundären Fixationsphase bildet sich dann eine stabile Verbindung durch "Verzahnung" des heranwachsenden Knochens mit der strukturierten Oberfläche. Die so erzeugten Rauhigkeiten liegen typischerweise im Bereich von 50 bis 150 µm und haben stochastische Eigenschaften, wodurch die Klemmkräfte in allen Beanspruchungsrichtungen in etwa gleich sein werden. So wird die primäre Haltekraft von Hüftpfannen mit derart strukturierten Oberflächen in etwa in der gleichen Grössenordnung wie Verklemmkraft oder die Extraktionskraft sein.

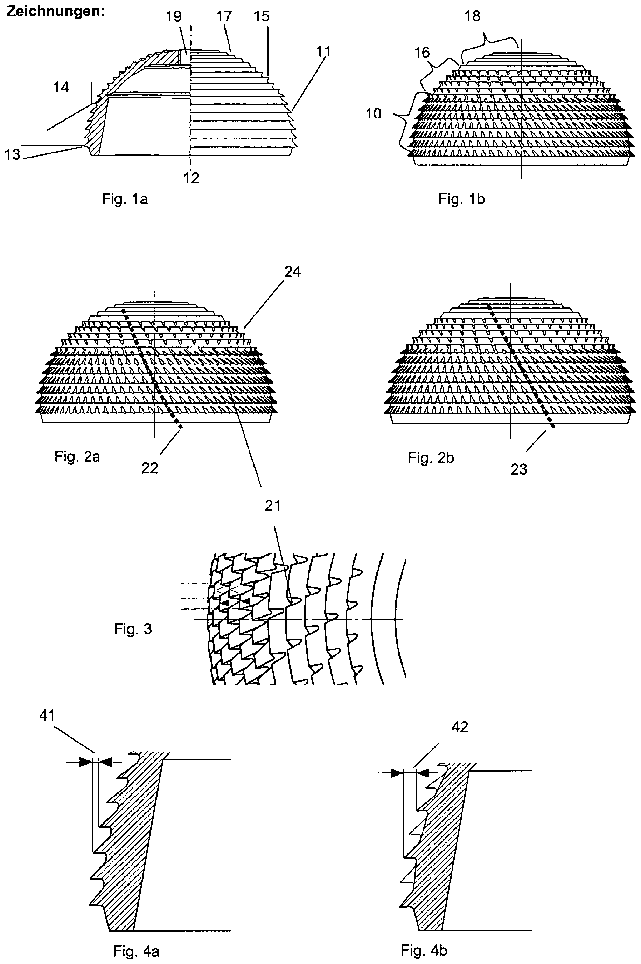

Alternativ werden Oberflächen von Verankerungsschalen, welche zur Verankerung im sphärisch gefrästen Knochenlager mittels Verklemmung bestimmt sind, mit gerichteten Eigenschaften erzeugt. Durch eine gezielte Gestaltung der Oberflächenstruktur werden gerichtete Eigenschaften für die Haltekraft erzeugt. Ein Beispiel hierfür ist die in der Schrift WO 02/064066 beschriebene zahnartige Struktur, welche durch ein zerspanendes Verfahren erzeugt wird. Konkret wird mittels einer Überlagerung von einem links- und rechtsdrehenden Gewindezerspanverfahren eine pyramidenförmige Zahnstruktur erzeugt. Bei den in diesem Verfahren generierten pyramidenförmigen Zähnen ist die steile Zahnflanke senkrecht zur Bauteilachse zum Pfannenäquator hin orientiert. Herstellungsbedingt sind die Zähne aber gegenüber dem knöchernen Bett in Auszugsrichtung nicht widerhakenförmig hinterschnitten. Zahntiefe nimmt beginnend am Äquator in Richtung Pol beständig ab, damit eine wirksame Zahnbreite erhalten werden kann. Ohne eine Verringerung der Zahntiefe würde bei der beschriebenen Zahngeometrie die Zahnbreite polwärts sehr schnell abnehmen, d.h. die Zähne würden sehr spitz werden. Gemäss den beschriebenen Ausführungen dieser Erfindung sind die Zahnreihen, gemäss z.B. Fig 2,4 und 5 entlang eines Längenkreises, d.h. entlang einer Linie von Pfannenpol senkrecht auf den Äquator in einer Reihe angeordnet. Dieser Makrostruktur wird mit einer Rauhigkeit überlagert, welche im Bereich von 4-10 µm liegt.

In der Schrift EP 0 639 356B1 ist eine hemisphärische Pfanne, ebenfalls mit einer geometrisch bestimmten Makrostruktur und das Verfahren der Struktur beansprucht. In zwei Verfahrensschritten werden zunächst in einer Drehbearbeitung konzentrischen Schneiden erzeugt, aus welchen in der folgenden Umformung mittels Prägestempel widerhakenförmigen Schuppen ausgeformt werden. Die Widerhakenförmige Struktur dehnt sich über den Klemmbereich der Pfanne aus, d.h. den Bereich, welcher nach dem Einschlagen in das untermassige Knochenbett sich unter radialer Spannung befindet. Im polnahen Bereich tritt geometriebedingt keine Radialspannung mehr auf. In diesem Bereich ist die Oberfläche mit feinen, nicht verzahnten Rillen zur Abstützung gegenüber dem Knochen ausgeführt. Die widerhakenförmige Struktur im Klemmbereich hat sich seit Mitte der 90-er Jahre langjährig klinisch besonders bewährt. Auch diese widerhakenförmigen Schuppen sind herstellungsbedingt entlang eines Längenkreises, d.h. entlang einer Linie von Pfannenpol senkrecht auf den Äquator in einer Reihe angeordnet. Auch diese Makrostruktur ist mit einer Rauhigkeit überlagert, welche im Bereich von 4-10 µm liegt. Eine sehr ähnliche Struktur wird in der Schrift US6231612 beansprucht, wobei die widerhakenförmige Zahnstruktur auch hier durch einen zerspanenden Herstellungsprozess erzeugt wird. Auch diese Struktur dehnt sich nur über den Klemmbereich der Pfanne aus. Wie aus der Beschreibung und den Figuren 2 und 3 ersichtlich sind auch diese Zahnreihen entlang einer Linie von Pfannenpol senkrecht auf den Äquator d.h. in einer Reihe angeordnet. Auch bei dieser Ausführung nimmt die Zahntiefe, beginnend vom Äquator in Richtung Pol, um eine passende wirksame Zahnbreite zu erhalten beständig ab.

Die FR2897528 wird als nächstkommender Stand der Technik angesehen sie zeigt jedoch insbesondere eine Überdeckung der aufeinanderfolgenden Zähne in Äquatornähe und zeigt keine Widerhakenform der Zähne.

Alle beschriebenen Oberflächengestaltungen mit Zahnstrukturen im Klemmbereich der Verankerungsschale weisen auf Grund der Richtungsabhängigkeit der Zahnformen richtungsabhängige Haltekräfte im Knochen auf. Die Steile Zahnflanke oder die "Widerhakenrichtung" ist immer zum Pfanneäquator hin orientiert und die flache Zahnflanke immer zur Pol hin orientiert. Gegen die steile Zahnflanke, oder gegen die Widerhaken- Richtung sind die Haltekräfte deutlich höher, als zur flachen Zahnflanke hin. Damit weisen diese Pfannen bei gleicher Setzkraft höhere Haltekräfte oder bei gleicher erwarteter Haltekraft geringere Setzkräfte auf.

Bei der Implantation von hemisphärischen Pfannen mit den beschriebenen Zahnstrukturen, welche in einer Reihe senkrecht zum Äquator angeordnet sind, wird die Knochenstruktur durch die in Reihe folgenden Zähne beim Einschlagvorgang furchenartig vorgeformt und der folgende Zahn kann nur mit der Differenz der Zahnhöhe zum nächsten polwärts angeordneten Zahn mit dem Knochen interagieren. Damit wird, je nach Lage des Zahnes auf der Halbschale nur ein Teil der Zahnhöhe für die Interaktion mit dem Knochen wirksam. Je näher sich die Zähne am Äquator befinden, desto geringer wird dadurch die wirksam interagierende Zahnhöhe. In der Äquatorregion ist dies von besonderer Bedeutung, da dort die grösste Klemmkraft herrscht, aus welcher die Rückhaltekraft d.h. die Primärstabilität der implantierten Hüftschale entsteht. Dieser Effekt tritt bei allen Zahnstrukturen auf, welche in einer Reihe, senkrecht zum Äquator, d.h. in Einschlagrichtung der Verankerungsschale angeordnet sind.

Aufgabe der vorliegenden Erfindung ist die Erzeugung einer erhöhten Rückhaltekraft der hemisphärischen Verankerungsschale mittels eines neuen Zahnmusters gegenüber bestehenden. Dabei wird die vorteilhafte Hinterschneidung der Zähne im Klemmbereich der Verankerungsschale zur Erzeugung des Widerhakeneffektes erhalten und der Verlust an Rückhaltekraft durch sich gegenseitig beeinflussende Zahnreihen deutlich reduziert. Dies wird dadurch gelöst, dass bei der Herstellung mittels eines den metallischen Werkstoff zerspanenden Verfahrens eine entweder linksdrehende oder rechtsdrehende Bahnkurve des zahnerzeugenden Fräswerkzeugs so gewählt wird, dass im Klemmbereich der Verankerungsschale die Zähne von einer Zahnreihe zu polwärts folgenden exakt auf Lücke gesetzt werden. Auch bei dieser Struktur verringert sich die Zahntiefe, beginnend am Äquator in Richtung Pol, da sonst bei gleich bleibender Zähnezahl in Richtung Pol die Zahnbreite schnell sinkt und so die verankerungswirksame Breite der Zähne schnell abnimmt. Ab dem Klemmbereich in Richtung Pol ist die Oberfläche zunächst mit kleinen, nicht hinterschnittenen Zähnen zur Stabilisierung, welche keine Rückhaltefunktion ausweisen und anschliessend mit einem Rillenmuster zur axialen Abstützung gegenüber dem Knochen gestaltet. Für einen gegebene Durchmesser der Verankerungsschale, eine gegebene Zahnform und einen Zahnreihenabstand existieren exakt zwei symmetrische Bahnkurven, d.h. zwei Führungskurven für das Fräswerkzeug, welche alternativ zu wählen sind, für welche die Bedingung, dass die Zähne reihenweise im Wechsle auf Lücke stehen gegeben ist.

Im Folgenden wird die Erfindung an Hand von Ausführungsbeispielen, welche in Zeichnungen dargestellt sind erläutert.
Fig. 1a: Verankerungsschale im Halbschnitt nach dem ersten Bearbeitungsschritt, als Zwischenstand der Herstellung, mit zirkulären Schneiden welche im Klemmbereich hinterschnitten sind und welche senkrecht zur Bauteilrotationsachse angeordnet sind und Schneiden zwischen Klemmbereich und Pol, welche nicht hinterschnitten sind.
Fig. 1b: Verankerungsschale fertig hergestellt mit drei Bereichen für die Fixierung im knöchernen Lager, wobei der Klemmbereich und der Stabilisierungsbereich durch ein Fräsverfahren mit Zähnen versehen sind und der Abstützbereich mit zirkulären Rillen versehen ist.
Fig. 2a: Verankerungsschale mit Zahnmuster im Klemmbereich mit dreidimensionaler Bahnkurve für das Fräswerkzeug wodurch die Zähne von einer Reihe zu nächst folgenden exakt auf Lücke positioniert sind, mit Verankerungsrillen im Stabilisierungs- und Stützbereich.
Fig. 2b: Verankerungsschale mit Zahnmuster im Klemmbereich mit einfach gekrümmter Bahnkurve für das Fräswerkzeug wodurch die Zähne von einer Reihe zu nächst folgenden mit geringfügiger Überdeckung auf Lücke positioniert sind. Die Bahnkurve erscheint als Gerade, da die Krümmung senkrecht zur Betrachtungsebene verläuft.
Fig. 3: Verzahnung im Klemmbereich aus der Pol- Ansicht mit Zähnen, welche von einer Zahnreihe zur nächst folgenden Zahnreihe auf Lücke angeordnet sind.
Fig. 4a: Situation der Interaktion zwischen Zahn und Knochen im Klemmbereich bei Zähnen, wobei die Zähne entlang einer Linie vom Pfannenpol senkrecht auf den Äquator angeordnet sind.
Fig. 4b: Situation der Interaktion zwischen Zahn und Knochen im Klemmbereich bei Zähnen, welche von Zahnreihe zu Zahnreihe auf Lücke angeordnet sind.

Aus Fig. 1a und Fig. 1b ist ersichtlich, dass im Klemmbereich (10) der Verankerungsschale die einzelnen Zahnreihen an der Zahnflanke, welche in Richtung Äquator orientiert ist, hinterschnitten sind, d.h. einen Widerhakeneffekt bilden. Dies wird erreicht in einem ersten Bearbeitungsschritt Fig. 1a, in welchem durch ein klassisches Drehverfahren zirkuläre Schneiden (11) erzeugt werden, welche räumlich senkrecht zur Rotationsachse (12) der Verankerungsschale angeordnet sind. Diese Schneiden bestimmen neben dem wirksamen Rückhaltewinkel (13) auch den Abgleitwinkel gegenüber dem Knochen (14) der die Setzkräfte beim Einschlagen beeinflusst. Die Höhe der Schneiden entspricht der späteren Zahnhöhe und nimmt, beginnend vom Äquator in Richtung Pol beständig ab. Ab einer gewissen Position auf der Verankerungsschale, ab der keine radiale Klemmwirkung mehr entsteht, sind Schneiden deutlich kleinerer Grösse (15) ausgeführt, welche nicht hinterschnitten sind und welche, wie in Fig. 1b dargestellt, durch den späteren Fräsprozess eine spitze Form erhalten. Anschliessen an diesen Stabilisierungsbereich (16) sind auf der Oberfläche zirkuläre Rillen (17) eingearbeitet, welche den polnahen Abstützbereich (18) und die Struktur bis zum Pol der Schale bilden. Im Pol befindet sich eine Bohrung (19), welche mit einem Gewinde versehen ist. Durch diese Polbohrung kann bei der Implantation die Setztiefe ertastet werden, d.h. der verbleibende Abstand zwischen Knochen und dem Pol der Verankerungsschale. Diese Bohrung wird nach erfolgter Implantation mit einer Schraube dicht verschlossen, damit keine Abriebpartikel aus dem Spalt zwischen Verankerungsschale und Inlay in das knöcherne Lager der Pfanne gelangen können, was zu Osteolysen führen kann. Das Gewinde dieser Bohrung dient zusätzlich zur Aufnahme des Implantationswerkzeuges, welches zur Implantation fest mit der Verankerungsschale zu verbinden ist.

In Fig. 2a und 2b ist die Verzahnung der Verankerungsschale dargestellt, wobei die Zähne von einer Reihe zur nächst folgenden auf Lücke positioniert sind. Die Verzahnung wird durch einen Fräsvorgang erzeugt, wobei die Gestalt der Zahnlücken durch die Formgebung des zahnlückengenerierenden Fräswerkzeuges bzw. dessen Bahnkurve bestimmt ist. Mit der Form des Fräswerkzeuges wird auch die Form der Zahnflanken (21), welche den Zahn in Umfangsrichtung begrenzen definiert. Die Bahnkurve der Fräserspur kann entweder rechtsdrehend oder alternativ linksdrehend gewählt werden. Eine beidseitig orientierte Fräsbearbeitung, d.h. die Überlagerung einer rechtsdrehenden und einer dazu symmetrischen linksdrehenden Fräsbearbeitung ist ebenso möglich. Neben einem höheren Bearbeitungsaufwand führt die Überlagerung einer rechts- und linksdrehenden Fräsbearbeitung auch zu einer etwas anderen Zahnform, welche für die Interaktion mit dem Knochen nicht günstig ist, da die Zähne spitzer werden. Wünschenswert sind Zähne mit einer, bei gegebener Zahngrösse möglichst breiten Schneide.

Im Ausführungsbeispiel in Fig. 2a und 2b ist eine vom Äquator in Richtung Pol orientierte linksdrehende Bahnkurve (22, 23) dargestellt. Für die Herstellung einer Verzahnung mit exakt von einer Zahnreihe zur folgenden auf Lücke angeordneten Zähnen ist eine räumlich gekrümmten Bahnkurve nach der Form (22) notwendig, herzustellen durch ein Zerspanungsverfahren, d.h. ein Fräsverfahren mit fünf simultan angesteuerten Bewegungsachsen. Die Bahnkurve kann wie in Fig 2b dargestellt, für vereinfachte Fertigungsverfahren, d.h. für Verfahren, wo drei simultan angesteuerte Bewegungsachsen zur Verfügung stehen, durch eine einfach gekrümmte Kurve, dargestellt als projizierte Gerade (23) angenähert werden, wodurch, abhängig vom Ort zwischen Äquator und Pol, eine leichte Abweichung, d.h. eine leichte Überdeckung der Zähne entsteht. Bei den für Hüftgelenke üblichen Durchmessern von Verankerungsschalen liegt diese Überdeckung im Bereich kleiner 0.2 mm, wobei für die einzelnen Grössen unterschiedliche Steigungen der projizierten Geraden, d.h. der ebenen Steigung der einfach gekrümmten Kurve resultieren.

Dargestellt sind die Bahnkurven für eine Ausführung von Verankerungsschale mit streng hemisphärischer Geometrie. Für Verankerungsschalen anderer Geometrie, z.B. solche mit abgeflachtem Pol, oder mit mehreren Radienzügen, hat diese Bahnkurve bei gleichem Durchmesser des Äquators einen anderen Verlauf. Wird die Bahnkurve rechtsdrehend, d.h. spiegelbildlich zur Achse ausgeführt, ergibt sich ein analoges Ergebnis. Die wirksame mit dem Knochen interagierende Zahntiefe, bzw. Zahnfläche wird dadurch nicht beeinflusst.

Um den patientenspezifischen Anforderungen entsprechen zu können, müssen innerhalb des Implantatsystems Verankerungsschalen mit unterschiedlichen Grössen zur Verfügung stehen. Unbeachtet der Grösse der Verankerungsschale hat sich, für die Verankerung gegenüber dem Knochen, eine Zahngrösse in einem gewissen Spektrum von 0.5 bis 2 mm als vorteilhaft erwiesen.

Da in allen Zahnreihen, beginnend am Äquator in Richtung Pol, eine konstante Zähnezahl gegeben ist, variiert die Zahngrösse der einzelnen Zahnreihen. Äquatornahe Zähne sind grösser, als polnahe. Um bei allen Grössen von Verankerungsschalen Zähne mit vergleichbarer Grösse erzeugen zu können, werden die einzelnen Grössen von Verankerungsschalen mit unterschiedlicher Zähnezahl am Umfang gestaltet. Grössere Verankerungsschalen haben eine grössere Zähnezahl als kleinere Verankerungsschalen.

Im Stabilisierungsbereich (16) werden durch den Fräsvorgang kleinere, nicht hinterschnittene Zähne (24) erzeugt. Diese Zähne dringen bei der Impaktion der Verankerungsschale in den Knochen ein und tragen so zur Stabilisierung gegenüber Kippung bei. In der Knochenanwachsphase bilden diese Zahnstrukturen mechanische Reizpunkte, an welchen das Knochenwachstum mit Vorzug stattfindet. Die Rillen im polbenachbarten Abstützbereich (17) bleiben als Drehrillen unverändert erhalten und werden nicht verzahnt.

Fig. 3 als vergrösserte Ansicht der äquatornahen Verzahnung aus der Polansicht betrachtet verdeutlicht das Resultat der Zahnanordnung mit Zähnen, welche von einer Reihe zu nächst folgenden exakt auf Lücke positioniert sind.

In Fig. 4a und 4b ist die Interaktion zwischen einem Zahn in Klemmbereich (10) und dem Folgezahn für die Ausführung gemäss dem Stand der Technik mit Zähnen in einer Linie (4a) und für die hier beanspruchte Ausführung mit Zähnen auf Lücke (4b) dargestellt. Es ist erkennbar, dass die wirksame, mit dem Knochen interagierende Zahntiefe (41) deutlich grösser ist im Falle der auf Lücke gesetzten Zahnreihen (42). Das Ausmass des Unterschiedes ist abhängig von der Krümmung am betrachteten Ort, welche abhängig ist von dem Durchmesser der Verankerungsschale, der Ausführung als Einradien- oder Mehrradienkonzept. Je grösser die Krümmung am betrachteten Ort, desto grösser die mit dem Knochen interagierende Zahntiefe und desto grösser ist der Unterschied (42). Da die mit dem Knochen interagierende Zahnfläche im Falle der auf Lücke gesetzten Zähne deutlich grösser ist, im Vergleich zu den in Linie angeordneten Zähne, ist die Rückhaltekraft im Falle der auf Lücke angeordneten Zähne deutlich grösser. Gegenüber den in einer Linie angeordneten Zahnstrukturen weist eine Verankerungsschale eine höhere Primärstabilität, als Voraussetzung für das Knochenanwachsen für die dauerhafte Fixierung im Knochen auf.

## Patentansprüche

1. Verankerungsschale für eine künstliche Hüftpfanne mit halbkugelförmiger oder nahezu halbkugelförmiger Außengeometrie, welche auf der Außenseite der Halbschale eine Zahnstruktur trägt, die ringförmig zur Bauteilachse (12) angeordnet ist,
wobei Zahnschneiden der Zähne räumlich senkrecht zur Bauteilachse (12) angeordnet sind,
wobei eine äquatorwärts orientierte Zahnflanke der einzelnen Zähne in einem äquatornahen Klemmbereich (10) der Verankerungsschale mit der Bauteilachse (12) einen Winkel von kleiner als neunzig Grad bildet, so dass zum Äquator hin ein Widerhakeneffekt entsteht,
wobei eine polwärts orientierten Zahnflanke der einzelnen Zähne im Klemmbereich (10) der Verankerungsschale mit der Bauteilachse (12) einen Winkel von mehr als einhundertzehn Grad bildet,
wobei eine Bahnkurve (22, 23) eines erzeugenden Werkzeuges, welches Zahnlücken erzeugt, einfach gekrümmt und damit eine räumlich projizierte Gerade ist und so gestaltet ist, dass die Zähne von einer Zahnreihe zur folgenden auf Lücke angeordnet sind, und die Zahnschneiden zweier vom Äquator in Richtung Pol aufeinander folgender Zähne sich nicht überdecken oder maximal um 0.2 mm.

2. Verankerungsschale nach Anspruch 1, **dadurch gekennzeichnet, dass** die Bahnkurve (22) des Werkzeuges, welches die Zahnlücken erzeugt, eine räumlich gekrümmte Kurve ist und so gestaltet ist, dass die Zähne von einer Zahnreihe zur folgenden Zahnreihe exakt auf Lücke angeordnet sind.

3. Verankerungsschale nach Anspruch 1 oder 2, **dadurch gekennzeichnet, dass** die Zahnstruktur mit auf Lücke angeordneten Zähnen und mit Widerhakengeometrie sich ab der Äquatorregion maximal auf der halben Strecke zwischen Äquator und Pol ausdehnt.

4. Verankerungsschale nach Anspruch 1 oder 2, **dadurch gekennzeichnet, dass** die Außengeometrie exakt einer Halbkugel entspricht.

5. Verankerungsschale nach Anspruch 1 oder 2, **dadurch gekennzeichnet, dass** die Außengeometrie einer am Pol abgeflachten Halbkugel entspricht.

6. Verankerungsschale nach Anspruch 1 oder 2, **dadurch gekennzeichnet, dass** sich im Pol eine Bohrung (19) befindet, welche mit einem Gewinde versehen ist.

7. Verankerungsschale nach Anspruch 1 oder 2, **dadurch gekennzeichnet, dass** die Schale in der Äquatorregion mit einem Rand abschließt, welcher glatt gestaltet ist und eine Breite von 0.5 bis 4 mm aufweist.

8. Verankerungsschale nach Anspruch 1 oder 2, **dadurch gekennzeichnet, dass** die Schale exakt eine Halbschale ist und damit exakt am Äquator endet.

9. Verankerungsschale nach Anspruch 1 oder 2, **dadurch gekennzeichnet, dass** die Schale größer als eine Halbschale ist und damit über den Äquator hinausreicht.

10. Verankerungsschale nach Anspruch 1 oder 2, **dadurch gekennzeichnet, dass** die Schale kleiner als eine Halbschale ist und damit vor dem Äquator endet.

11. Verankerungsschale nach Anspruch 1 oder 2, **dadurch gekennzeichnet, dass** sich im Innern der Schale eine zum Äquator hin offene konische Aufnahme mit einem gesamten Winkel zwischen 15 und 22 Winkelgraden befindet.

12. Verankerungsschale nach Anspruch 1 oder 2 und Anspruch 11, **dadurch gekennzeichnet, dass** sich im Innern der Schale eine zur Bauteilachse (12) senkrecht angeordnete Ringnut befindet.

## Claims

1. Anchoring shell for an artificial acetabulum, having a hemispherical or virtually hemispherical external geometry, which bears a toothed structure on the outer side of the half-shell, said toothed structure being arranged annularly with respect to the component axis (12),
wherein tooth cutting edges of the teeth are arranged spatially perpendicularly to the component axis (12),
wherein a tooth flank, oriented towards the equator, of the individual teeth forms an angle of less than ninety degrees with the component axis (12) in a clamping region (10), close to the equator, of the anchoring shell, such that a barb effect arises in the direction of the equator, wherein a tooth flank, oriented towards the pole, of the individual teeth forms an angle of more than one hundred and ten degrees with the component axis (12) in the clamping region (10) of the anchoring shell,
wherein a path curve (22, 23) of a producing tool which produces tooth gaps is single-curved and thus is a spatially projected straight line, and is formed such that the teeth are arranged in a staggered manner from one row of teeth to the next, and the tooth cutting edges of two teeth that follow one another from the equator in the direction of the pole do not overlap or overlap at most by 0.2 mm.

2. Anchoring shell according to Claim 1, **characterized in that** the path curve (22) of the tool which produces the tooth gaps is a spatially arched curve and is formed such that the teeth are arranged in an exactly staggered manner from one row of teeth to the next row of teeth.

3. Anchoring shell according to Claim 1 or 2, **characterized in that** the tooth structure with teeth arranged in a staggered manner and with a barbed geometry stretches from the equator region at most to half the distance between the equator and pole.

4. Anchoring shell according to Claim 1 or 2, **characterized in that** the external geometry corresponds exactly to a hemisphere.

5. Anchoring shell according to Claim 1 or 2, **characterized in that** the external geometry corresponds to a hemisphere flattened at the pole.

6. Anchoring shell according to Claim 1 or 2, **characterized in that** the pole contains a bore (19) which is provided with a thread.

7. Anchoring shell according to Claim 1 or 2, **characterized in that** the shell terminates in the equator region with a rim which is formed in a smooth manner and has a width of 0.5 to 4 mm.

8. Anchoring shell according to Claim 1 or 2, **characterized in that** the shell is exactly a half-shell and thus ends exactly at the equator.

9. Anchoring shell according to Claim 1 or 2, **characterized in that** the shell is larger than a half-shell and thus extends beyond the equator.

10. Anchoring shell according to Claim 1 or 2, **characterized in that** the shell is smaller than a half-shell and thus ends before the equator.

11. Anchoring shell according to Claim 1 or 2, **characterized in that** a conical receptacle that is open towards the equator and has an overall angle of between 15 and 22 angle degrees is located inside the shell.

12. Anchoring shell according to Claim 1 or 2 and Claim 11, **characterized in that** an annular groove that is arranged perpendicularly to the component axis (12) is located inside the shell.

## Revendications

1. Cupule d'ancrage pour un cotyle de hanche artificielle ayant une géométrie extérieure présentant la forme d'une demi-sphère ou sensiblement d'une demi-sphère, qui comporte sur la face extérieure de la demi-cupule une structure dentée qui est disposée sous forme d'anneaux autour de l'axe structural (12), tandis que les tranchants des dents sont disposés spatialement perpendiculairement à l'axe structural (12),
tandis qu'un flanc des dents orienté dans la direction équatoriale des dents individuelles forme, dans un domaine de blocage (10) proche de l'équateur de la cupule d'ancrage, un angle inférieur à quatre-vingt-dix degrés avec l'axe structural (12), de telle sorte qu'il se produit un effet d'accrochage vis-à-vis de l'équateur,
tandis qu'un flanc des dents orienté dans la direction polaire des dents individuelles forme dans le domaine de blocage (10) proche de l'équateur de la cupule d'ancrage, un angle supérieur à cent-dix degrés avec l'axe structural (12),
tandis qu'une portion de trajectoire (22, 23) d'un outil de fabrication qui produit les vides de dents est simplement courbée et ainsi projetée dans l'espace selon une droite et est ainsi configurée de telle sorte que les dents d'une rangée de dents sont réparties en correspondance avec des vides de la suivante, et les tranchants des dents de deux en deux à partir de l'équateur en direction du pôle ne se chevauchent pas avec les dents suivantes ou au maximum sur 0,2 mm.

2. Cupule d'ancrage selon la revendication 1, **caractérisée en ce que** la portion de trajectoire (22) de l'outil qui produit les vides entre les dents est une courbe gauchie dans l'espace et est configurée de telle sorte que les dents d'une rangée de dents sont réparties exactement sur des vides en direction de la rangée de dents suivante.

3. Cupule d'ancrage selon la revendication 1 ou 2, **caractérisée en ce que** la structure dentée se déploie avec des dents réparties sur des vides et avec une géométrie des agrippements, à partir de la région équatoriale, au maximum sur la moitié de la distance entre l'équateur et le pôle.

4. Cupule d'ancrage selon la revendication 1 ou 2, **caractérisée en ce que** la géométrie extérieure correspond exactement à une demi-sphère.

5. Cupule d'ancrage selon la revendication 1 ou 2, **caractérisée en ce que** la géométrie extérieure correspond à une demi-sphère aplatie au pôle.

6. Cupule d'ancrage selon la revendication 1 ou 2, **caractérisée en ce qu'**il se trouve au pôle un alésage (19) qui est muni d'un filetage.

7. Cupule d'ancrage selon la revendication 1 ou 2, **caractérisée en ce que** la cupule se termine dans la région équatoriale par un bord qui présente une configuration plate et une largeur de 0,5 à 4 mm.

8. Cupule d'ancrage selon la revendication 1 ou 2, **caractérisée en ce que** la cupule est exactement une demi-cupule et se termine ainsi exactement à l'équateur.

9. Cupule d'ancrage selon la revendication 1 ou 2, **caractérisée en ce que** la cupule est plus grande qu'une demi-cupule et dépasse ainsi de l'équateur.

10. Cupule d'ancrage selon la revendication 1 ou 2, **caractérisée en ce que** la cupule est plus petite qu'une demi-cupule et se termine ainsi avant l'équateur.

11. Cupule d'ancrage selon la revendication 1 ou 2, **caractérisée en ce qu'**il se trouve à l'intérieur de la cupule un logement conique ouvert en direction de l'équateur avec un angle total entre 15 et 22 degrés d'angle.

12. Cupule d'ancrage selon la revendication 1 ou 2 et la revendication 11, **caractérisée en ce qu'**il se trouve à l'intérieur de la cupule une gorge circulaire agencée perpendiculairement à l'axe structural (12).
